(19) Europäisches Patentamt
European Patent Office
Office européen des brevets

(11) Publication number: **0 202 854 A2**

# (12) EUROPEAN PATENT APPLICATION

(21) Application number: 86303646.3

(22) Date of filing: 14.05.86

(51) Int. Cl.[4]: C 07 F 9/50
C 07 H 23/00, A 61 K 31/28
A 61 K 31/71

(30) Priority: 20.05.85 US 736003
20.05.85 US 736001
23.12.85 US 812230

(43) Date of publication of application:
26.11.86 Bulletin 86/48

(84) Designated Contracting States:
AT BE CH DE FR GB IT LI LU NL SE

(71) Applicant: SMITHKLINE BECKMAN CORPORATION
One Franklin plaza
Philadelphia Pennsylvania 19103(US)

(72) Inventor: Hill, David Taylor
109 Magnolia Place
North Wales Pennsylvania 19454(US)

(72) Inventor: Johnson, Randall Keith
71 Llanfair Circle
Ardmore Pennsylvania 19003(US)

(74) Representative: Waters, David Martin, Dr. et al,
Smith Kline & French Laboratories Ltd. Patent
Department Mundells
Welwyn Garden City Hertfordshire AL7 1EY(GB)

## (54) Phosphine gold compounds.

(57) Compounds of formula (I) and (III)

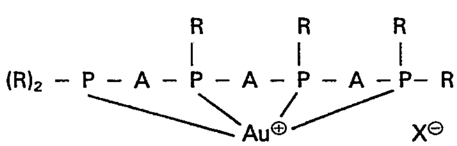

(I)

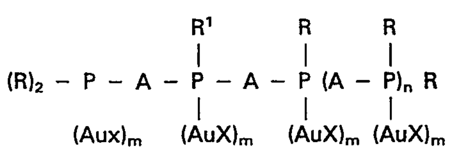

(III)

wherein R is phenyl; $R^1$ is phenyl or $-AP(AuX)(R)_2$; A is the same and is a straight or branched alkanediyl chain of from one to six carbon atoms; n is 0 or 1; m is the same and is 0 or 1; and X is the same and is halo or thiosugar; provided that when n is 1, $R^1$ is phenyl; compositions containing them and their use in therapy for inhibiting the growth of animal tumor cells.

EP 0 202 854 A2

Croydon Printing Company Ltd.

56303663



TITLE

# PHOSPHINE GOLD COMPOUNDS

## BACKGROUND OF THE INVENTION

This invention relates to novel phosphine gold compounds which have tumor cell growth-inhibiting activity, pharmaceutical compositions containing an effective tumor cell growth-inhibiting amount of a phosphine gold compound, and a method for treating tumor cells sensitive to such a compound by administering a tumor cell growth-inhibiting amount of a phosphine gold compound to a host animal afflicted by such tumor cells.

Various references including Clark et al., U.S. Patent 3,364,273, issued January 16, 1968; King et al., J. Am. Chem. Soc., 91 (18), 5191-26 (1969); King et al., J. Am. Chem. Soc., 93 (17), 4158-66 (1971); King et al., Inorg. Chem., 10(9), 1851-67 (1971); King, Acc. Chem. Res., 5, 177-85 (1972) and King et al., U.S. Patent 3,657,298, issued April 18, 1982 disclose 1,1,4,7,10,10-hexaphenyl-1,4,7,10-tetraphosphodecane.

Various references including Bartish, U.S. Patent 4,102,920, issued July 25, 1978, and Antberg et al. Inorg. Chem., 23(5), 4170-4174 (1984), disclose bis(2-diphenylphosphinoethyl)phenyl phosphine and/or bis(2-diphenylphosphinopropyl)phenyl phosphine.

Various references including Hebb et al., U.S. Patent 4,361,707 issued August 3, 1981, disclose tris(diphenylphosphinoethyl)phosphine. Deutsch et al., U.S. Patent 4,387,087, issued June 7, 1983, disclose the use of tris(diphenylphosphinoethyl)phosphine in a method of imaging organs with technetium radiopharmaceuticals.

Cooper et al., Inorg. Chim. Acta, 65(5), L185-186 (1982), disclose the synthesis and X-ray structure of trichloro1,1,1-(diphenylphosphinomethyl)ethane[trigold(I)].

Several references including Hewertson et al., J. Chem. Soc., 1490-1495 (1962) and Safridis et al., U.S. Patent 3,445,540, issued May 20, 1969, disclose μ-[1,1,1-tris(diphenylphosphinomethyl)ethane.

Cariati et al., Inorg. Chim. Acta, 1(2), 315-18 (1967), and Bates et al., Inorg. Chim. Acta, 81(2), 151-156 (1984) disclose bis[1,2-bis(diphenylphosphino)ethane]gold(I) chloride.

Struck et al., J. Med. Chem., 9, 414-417 (1966), disclose cytotoxic activity for 1,2-bis(diphenylphosphino)ethane.

None of the aforementioned references disclose or suggest the compounds, pharmaceutical compositions and/or methods of treatment of the subject invention.

## SUMMARY OF THE INVENTION

This invention relates to phosphine gold(I) compounds of the formula:

or

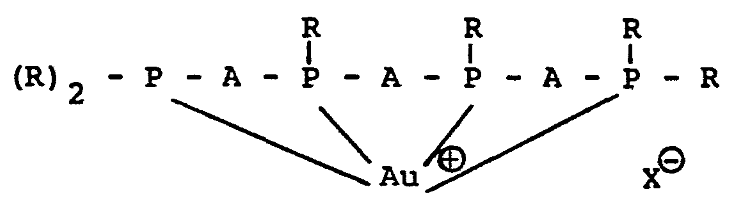

Formula (I)

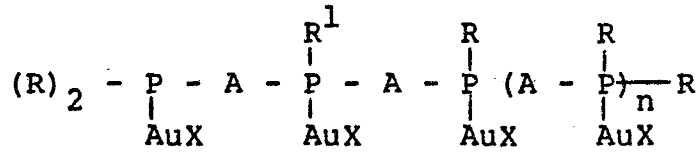

Formula (II)

wherein:

R is phenyl

$R^1$ is phenyl or $-AP(AuX)(R)_2$

A is the same and is a straight or branched alkanediyl chain of from one to six carbon atoms;

n is 0 or 1; and

X is the same and is (i) halo for compounds of Formula (I) and (ii) halo or thiosugar for compounds of Formula (II);

provided that when n is 1, $R^1$ is phenyl. When X is thiosugar, the attachment of X to the gold atom is through the sulfur atom of the thiosugar.

This invention also relates to a pharmaceutical composition which comprises an effective, tumor cell growth- inhibiting amount of an active ingredient and an inert, pharmaceutically acceptable carrier or diluent, wherein said composition is useful for inhibiting the growth of animal tumor cells sensitive to the active ingredient, and wherein the active ingredient is a compound of the formula:

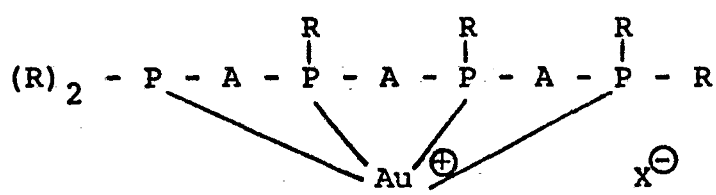

or

Formula (I)

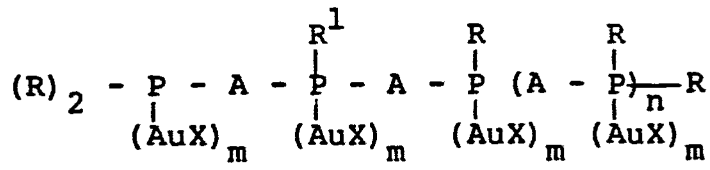

Formula III

wherein:

R is phenyl

$R^1$ is phenyl or $-AP(AuX)(R)_2$

A is the same and is a straight or branched alkanediyl chain of from one to six carbon atoms;

n is 0 or 1;

m is the same and is 0 or 1; and

X is the same and is (i) halo for compounds of Formula (I) and (ii) halo or thiosugar for compounds of Formula (II);

provided that when n is 1, $R^1$ is phenyl; and a pharmaceutically acceptable carrier.

When X is thiosugar, the attachment of X to the gold atom is through the sulfur atom of the thiosugar.

Another aspect of this invention relates to a method of inhibiting the growth of animal tumor cells sensitive to a compound of Formula (I) or Formula (III) which comprises administering to an animal afflicted with said tumor cells, an effective, tumor cell growth-inhibiting amount of a compound of Formula (I) or Formula (III).

## DETAILED DESCRIPTION OF THE INVENTION

It is important to note that all of the compounds of Formula (II) are encompassed within the scope of Formula (III).

By the term "thiosugar" is meant any 1-thioaldose. Examples of such thiosugars include 1-thioglucose, 1-thiogalactose, 1-thiomannose,

1-thioribose, 1-thiomaltose, 1-thiofucose, tetra-O-acetyl-1-thioglucose, tetra-O-acetyl-1-thiomannose, tetra-O-acetyl-1-thiogalactose, tri-O-acetyl- 1-thioribose, hepta-O-acetyl-1-thiomaltose and tri-O-acetyl- 1-thiofucose.

All the compounds of Formula (I) and Formula (III) are either available commercially or can be prepared by methods available to one skilled in the art.

Generally, the compounds of Formula (III) wherein X is chloro and m is 1 can be prepared by reacting the appropriate compound of Formula (III) wherein M is 0 with chloroauric acid tetrahydrate reduced by treatment with thiodiglycol. All the necessary compounds of Formula (III) wherein M is 0 are available from commercial sources, for example, Strem Chemicals, Inc., Newburyport, Massachusetts.

The compounds of Formula (III) wherein X is bromo and m is 1 are prepared by reacting the appropriate compound of Formula (III) wherein m is 0 with bromoauric acid hydrate, (which is commercially available, for example from Strem Chemicals, Inc., Newburyport, Massachusetts), reduced by treatment with thiodiglycol. Alternatively, compounds of Formula (III) wherein X is bromo and m is 1 are prepared by treatment of the corresponding compounds of Formula (III) wherein X is chloro and m is 1 with sodium bromide in an appropriate non-reactive organic solvent, such as aqueous ethanol or DMF.

The compounds of Formula (III) wherein X is iodo and m is 1 are prepared by treatment of the corresponding compounds of Formula (III) wherein X is chloro or bromo and m is 1 with sodium iodide in an appropriate non-reactive organic solvent, such as acetone.

All the necessary compounds of Formula (III) wherein m is 0 are available from commercial sources, for

example, from Strem Chemicals, Inc., Newburyport, Massachusetts.

To prepare the Formula (III) compounds wherein X is thiosugar and m is 1, the corresponding Formula (III) compound wherein X is chloro and m is 1 is reacted with a thiosugar source, for example, sodium thioglucose. The necessary thiosugar source is available from commercial sources, for example, from Sigma Chemicals Co., St. Louis, Missouri.

Generally, the compounds of Formula (I) wherein X is chloro or bromo can be prepared by reacting three moles of the appropriate compound of Formula (III) wherein $R^1$ is phenyl, m is 0 and n is 1, with one mole of the appropriate compound of Formula (III) wherein $R^1$ is phenyl, n is 1 and m is 1, in a non-reactive organic solvent. Alternatively, the compounds of Formula (I) wherein X is bromo are prepared by reacting the appropriate compound of Formula (I), wherein X is chloro, with sodium bromide in an appropriate organic solvent, such as aqueous ethanol or DMF.

The compounds of Formula (I) wherein X is iodo are prepared by reacting the appropriate compound of Formula (I) wherein X is chloro or bromo with sodium iodide in an appropriate organic solvent, such as acetone.

The compounds of Formula (I) and Formula (III) have tumor cell growth-inhibiting activity which has been demonstrated in at least one animal tumor model.

P388 lymphocytic leukemia is currently the most widely used animal tumor model for screening for antitumor agents and for detailed evaluation of active compounds. This tumor system is widely accepted as an antitumor agent screening tool because it is sensitive to virtually all of the clinically active antineoplastic agents; quantitative and reproducible; amenable for large-scale screening; and predictive for activity in other animal tumor models.

Drugs that are highly active in intraperitoneal (ip) P388 leukemia are generally active in other tumor models as well. The antitumor activity of the compounds of Formula (I) and Formula (III) is demonstrated in the P388 leukemia mouse model employing the following protocol:

> $10^6$ P388 leukemia cells are inoculated ip in $B6D2F_1$ mice. Twenty-four hours later, if the tumor inoculum proves to be free of bacterial contamination (as determined by 24 hours incubation in thioglycollate broth), animals are randomized into groups of 6 and housed in shoebox cages. The compound to be evaluated is dissolved in a minimal volume of either N,N- dimethylacetamide (DMA) or 95% ethanol (depending upon solubility). An equal volume of saline is added; if the drug comes out of solution an equal volume of polyethoxylated castor oil is added and then saline qs to a concentration such that the desired dose is delivered in 0.5 ml. The final concentration of DMA, ethanol and polyethoxylated castor oil is $\geq$ 10 percent. Dilutions for lower doses are made with saline so there is a decreasing proportion of organic solvents in the vehicle with decreasing dosage. These vehicles provide soluble formulations (or suspensions). Formulations are prepared immediately prior to injection. The compound is administered ip on Days 1 through 5 (i.e., treatment is initiated 24 hrs after tumor inoculation). Each experiment includes three groups of 6 animals as untreated controls and animals treated with a positive control, cisplatin, at two dose levels. Animals are weighed as a group on Days 1, 5 and 9 and average weight change ($\Delta$ wt.) is used as a reflection of toxicity. Each experiment also

includes an inoculum titration -- groups of 8 mice inoculated ip with $10^5$ to 10° P388 leukemia cells. The titration is used to calculate cell kill achieved by treatment with drugs. Animals are monitored daily for mortality and experiments are terminated after 45 days. The endpoint is median survival time (MST) and increase in lifespan (ILS) which is the percentage of increase in MST relative to untreated controls. Untreated controls inoculated ip with $10^6$ P388 leukemia cells generally survive for a median of 9 to 11 days. A drug is considered active if it produces $\geq$25 percent ILS.

A summary of the evaluation of a compound of Formula (I) in the *in vivo* ip P388 model is shown in the following Table A.

TABLE A

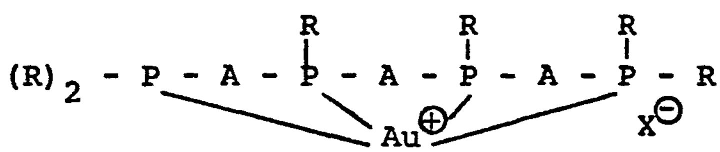

Formula (I)

| Compound Number | R | A | X | MTD[a] (mg/kg) | ILS (max)[b] (%) |
|---|---|---|---|---|---|
| 1 | phenyl | $(CH_2)_2$ | Cl | 3 | 40/65 |

[a] maximally tolerated dose for B62DF female mice on an ip qDX5 regimen.

[b] maximum increase in lifespan produced in mice bearing ip P388 leukemia (figures separated by slashes indicate data generated in separate experiments).

Based on the data set forth in Table A, a compound of Formula (I) showed significant antitumor activity in the <u>in vivo</u> ip P388 leukemia tumor assay.

A summary of the evaluation of several compounds of Formula (III) in the <u>in vivo</u> ip P388 model is shown in the following Table B.

TABLE B

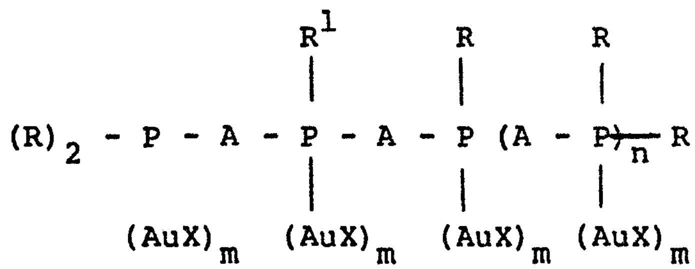

Formula III

| Cmpd. No. | $R^1$ | R | n | m | A | X | MTD[(a)] (mg/kg) | ILS (max)[(b)] % |
|---|---|---|---|---|---|---|---|---|
| 2 | phenyl | phenyl | 0 | 1 | $(CH_2)_2$ | Cl | 8 | 30/40 |
| 3 | -AP(AuX) $(R)_2$ | " | 0 | 1 | $(CH_2)_2$ | Cl | 8 | 45/45 |
| 4 | phenyl | " | 1 | 1 | $(CH_2)_2$ | Cl | 12 | 85/105/50/60 |
| 5 | phenyl | " | 1 | 1 | $(CH_2)_2$ | thio-glucose | 8 | 100/55 |
| 6 | phenyl | " | 0 | 0 | $(CH_2)_2$ | N/A | 32 | 80/50 |
| 7 | -AP(AuX) $(R)_2$ | " | 0 | 0 | $(CH_2)_2$ | N/A | 64 | 35/30 |
| 8 | phenyl | " | 1 | 0 | $(CH_2)_2$ | N/A | 64 | 25/40 |

(a) maximally tolerated dose for B6D2F female mice on an ip qDx5 regimen.

(b) maximum increase in lifespan produced in mice bearing ip P388 leukemia (figures separated by slashes indicate data generated in separate experiments).

The cytotoxic activity of Compound No. 1 of Table A and Compound No. 4 of Table B was evaluated <u>in vivo</u> using

B16 melanoma cells. In this system, groups of eight $B6D2F_1$ mice are inoculated ip with 0.5 ml of a 10% (w:v) brei of B16 melanoma prepared from pooled sc tumors excised at 14-21 days from C57Bl/6 donor mice. Daily treatment is begun 24 hours after tumor implantation and is continued daily for ten (10) days. The route of drug administration is ip. The mice are monitored daily for survival for sixty (60) days. Antitumor activity is assessed by prolongation of median survival time. An ILS of $\geq$25% indicates activity in this tumor model.

A summary of the results of the in vivo ip B16 melanoma assay is shown in Table C.

TABLE C

| Compound No. | MTD (mg/kg)[b] | ILS (%)[c] |
|---|---|---|
| 1[a] | 2 | 37 |
| 4[d] | 6 | 30/51 |

(a) see Table A for structure.
(b) maximally tolerated dose for $B6D2F_1$ mice on an ip qDx10 regimen.
(c) maximum increase in lifespan produced in mice bearing ip B16 melanoma.
(d) see Table B for structure.

Another chemosensitive tumor model is intraperitoneally (ip) implanted M5076 reticulum cell sarcoma in mice. In this system, B6D2F female mice are inoculated with 0.5 ml of a 10 percent (w:v) brei of M5076 prepared from pooled subcutaneous (sc) tumors excised at about 21 days from C57Bl/6 donors. Drugs are administered ip. Daily treatment is begun 24 hours after implantation and is continued for ten days. The treatment regimen for M5076 is more prolonged than for P388 because of the slower growth rate and longer control survival time of the M5076 tumor. A drug is considered active in this

tumor model if it produces $\geq$25% ILS.

The antitumor activity of Compound No. 1 of Table A and Compound No. 4 of Table B in the M5076 reticulum cell sarcoma tumor model is set forth in Table D.

TABLE D

| Compound No. | MTD (mg/kg)[b] | ILS (MAX) (%)[c] |
|---|---|---|
| 1[a] | 2 | 38 |
| 4[d] | 8 | 96 |

(a) see Table A for structure.
(b) maximally tolerated dose for B6D2F female mice on ip qDx10 regimen.
(c) maximum increase in lifespan produced in mice bearing ip M5076 reticulum cell sarcoma.
(d) see Table B for structure.

The pharmaceutical compositions of this invention comprise an effective tumor cell growth-inhibiting amount of a compound of Formula (I) or Formula (III) and an inert pharmaceutically acceptable carrier or diluent. These compositions are prepared in dosage unit form appropriate for parenteral administration.

Compositions according to the invention for parenteral administration include sterile aqueous or non-aqueous solutions, suspensions or emulsions. The composition may be in the form of a solution of the active ingredient in a minimal volume of dimethylacetamide or ethanol, for example 5% v/v, brought up to volume with peanut oil or normal saline solution. Polyethoxylated castor oil, for example 2 to 5% v/v, may also be used to solubilize the active ingredient. In addition, the composition may be in the form of a slurry with, for example, hydroxypropyl cellulose or other suitable suspending agent. As an emulsifying agent, lecithin for

example may be used. The composition may also be provided in the form of a sterile solid which can be dissolved in a sterile injectable medium immediately before use.

Freireich et al., Cancer Chemo. Rept., 50, 219-244 (1966), compared the quantitative toxicity of 18 anticancer drugs in six species after correcting the data to a uniform schedule of treatment for five consecutive days. This analysis demonstrated that mouse, rat, dog, human, monkey and man have essentially the same maximum tolerated dose (MTD) when compared on a basis of $mg/m^2$ of body surface area. The study suggested that Phase I clinical trials could be safely initiated at a dose one-third the animal MTD. The mouse was as useful as any other species in this regard on which to base the calculation. The appropriate therapeutically effective dose for any compound of the invention can therefore be determined readily by those skilled in the art from simple experimentation with laboratory animals, preferably mice.

It will be appreciated that the actual preferred dosages of the compounds of Formula (I) or Formula (III) used in the compositions of this invention will vary according to the particular site, host and disease being treated. The route of internal administration should be selected to ensure that an effective tumor cell growth-inhibiting amount of the compound of Formula (I) or Formula (III) contacts the tumor. Optimal dosages for a given set of conditions can be ascertained by those skilled in the art using conventional dosage determination tests in view of the above experimental data. For parenteral administration of a compound of Formula (I) the dose preferably employed is from about 3 to about 27 $mg/m^2$ of body surface per day for five days, repeated about every fourth week for four courses of treatment. For parenteral administration of a Formula (III) compound

the dose preferably employed is from about 5 to about 600 $mg/m^2$ of body surface per day for five days, repeated about every fourth week for four courses of treatment.

The method for inhibiting the growth of animal tumor cells sensitive to compound of Formula (I) or Formula (III) in accordance with this invention comprises administering to a host animal afflicted with said tumor cells, an effective tumor cell growth-inhibiting amount of a compound of Formula (I) or Formula (III).

## EXAMPLES

The following examples illustrate the chemical preparation of several compounds of Formula (I) and Formula (III) which are used in the compositions and methods of this invention and as such are not to be construed as limiting the scope thereof. All temperatures are in degrees Centigrade.

### EXAMPLE 1

### μ-[Bis(2-diphenylphosphinoethyl)phenylphosphine]tris-(chlorogold)

Chloroauric acid tetrahydrate (2.5 g, 6.07 mmole) in water (10 ml) was reduced upon addition of thiodiglycol (3.0 g, 24.6 mmoles) in water (10 ml)/methanol (60 ml) at 0°. A solution of bis(2-diphenylphosphinoethyl)phenylphosphine (1.08 g, 2.02 mmole), a Formula (III) compound obtained from Strem Chemicals Inc., Newburyport, Massachusetts, in chloroform (50 ml)/methanol (30 ml) was added and the mixture was stirred for several hours, and allowed to come to room temperature. Water was added and the product extracted with chloroform/methylene chloride, dried ($MgSO_4$), filtered and the solvent was removed *in vacuo*. The residue was slurried with chloroform, diluted with ethanol and the precipitate collected and dried to give 1.75 g (70%) of product, melting point (m.p.)

173-177°.

EXAMPLE 2

μ-[Tris(2-diphenylphosphinoethyl)phosphine]tetrakis-(chlorogold)

Chloroauric acid tetrahydrate (2.5 g, 6.06 mmole) in water (20 ml) was reduced upon addition of thiodiglycol (3.0 g, 24.6 mmole) in water (20 ml)/methanol (60 ml) at 0°. A solution of tris(diphenylphosphinoethyl)phosphine (1.02 g, 1.5 mmole), a Formula (III) compound obtained from Strem Chemicals Inc., Newburyport, Massachusetts, in chloroform (30 ml)/methanol (20 ml) was added, and the mixture was stirred for several hours. Water (200 ml) was added, and the mixture was extracted with chloroform. The chloroform extracts were dried ($MgSO_4$), filtered and the solvent was removed *in vacuo*. Methanol was added to the residue and the solution was cooled. The crystals were collected, dissolved in methylene chloride, and treated with activated carbon. Then methanol was added and the mixture was cooled. The resulting crystals were collected to give 2.1 g (86%), of the named Formula (III) product, m.p. 181-3°.

EXAMPLE 3

μ-[1,1,4,7,10,10-Hexaphenyl-1,4,7,10-tetraphosphodecane]-tetrakis(chlorogold)

Chloroauric acid tetrahydrate (1.87 g, 4.54 mmol) in water (20 ml) was reduced upon addition of thiodiglycol (3.0 g, 24.6 mmole) in methanol (60 ml)/water (20 ml) at 0°. 1,1,4,7,10,10-hexaphenyl-1,4,7,10-tetraphosphodecane (0.76 g, 1.13 mmole), a Formula (III) compound obtained from Strem Chemicals, Inc. Newburyport, Massachusetts, in chloroform (60 ml)/methanol (20 ml) was added and the mixture was stirred for several hours. The resulting precipitate was collected and dissolved in methylene

chloride. Then hexane was added and the mixture was cooled. Collection of successive crops gave 1.35 g (94%) of the named Formula (III) product, m.p. 184-186°.

EXAMPLE 4

μ-[1,1,4,7,10,10-Hexaphenyl-1,4,7,10-tetraphosphodecane]-tetrakis(1-thio-ß-D-glucopyranosato-S) gold

A mixture of sodium ß-D-thioglucose (0.97 g, 4.4 mmoles), obtained from Sigma Chemical Co., St. Louis, Missouri, and μ-[1,1,4,7,10,10-Hexaphenyl-1,4,7,10,-tetraphosphodecane] tetrakis(chlorogold) (1.6 g, 1 mmole), prepared as described in Example 3, in methanol (150 ml)/chloroform (150 ml)/water (20 ml) was stirred for 18 hours at ambient temperature, and the solvent was evaporated. The residue was washed with water, and then with acetone. The residue was then treated with boiling methanol and filtered, and the methanol was evaporated. Crystallization of the residue from acetone gave 1.5 g (67%) of the named Formula (III) product, m.p. 267-272°.

EXAMPLE 5

Using the procedure of Example 1, by reacting the appropriate Formula (III) compound wherein m is 0 and the appropriate haloauric acid tetrahydrate, reduced by treatment with thiodiglycol, the following compounds of Formula (III) wherein X is chloro or bromo are prepared, or by reacting the appropriate Formula (III) compound wherein X is chloro with sodium bromide in an appropriate organic solvent, such as aqueous ethanol or DMF, the following compounds of Formula (III) wherein X is bromo are prepared, and by reacting the appropriate compound of Formula (III) wherein X is chloro or bromo with sodium iodide in an appropriate organic solvent, such as acetone, the following compounds of Formula (III) wherein X is iodo are prepared:

a. μ -[bis(2-diphenylphosphinomethyl)phenylphosphine] tris-(chlorogold)

b. μ -[bis(2-diphenylphosphinopropyl)phenylphosphine] tris-(chlorogold)

c. μ -[bis(2-diphenylphosphinobutyl)phenylphosphine] tris-(chlorogold)

d. μ -[bis(2-diphenylphosphinopentyl)phenylphosphine] tris-(chlorogold)

e. μ -[bis(2-diphenylphosphinohexyl)phenylphosphine] tris-(chlorogold)

f. μ -[bis(2-diphenylphosphinoethyl)phenylphosphine] tris-(bromogold)

g. μ -[bis(2-diphenylphosphinoethyl)phenylphosphine] tris-(iodogold)

## EXAMPLE 6

Using the procedure of Example 4, by reacting the appropriate thioglucose source with the appropriate Formula (III) compound wherein X is chloro, prepared according to the procedure of Example 1 or 5, the following Formula (III) compounds wherein X is thioglucose are prepared:

a. μ -[bis(2-diphenylphosphinomethyl)phenylphosphine] tris-(1-thio-ß-D-glucopyranosato-S)gold

b. μ -[bis(2-diphenylphosphinoethyl)phenylphosphine] tris-(1-thio-ß-D-glucopyranosato-S)gold

c. μ -[bis(2-diphenylphosphinopropyl)phenylphosphine] tris-(1-thio-ß-D-glucopyranosato-S)gold

d. μ -[bis(2-diphenylphosphinobutyl)phenylphosphine] tris-(1-thio-ß-D-glucopyranosato-S)gold

e. μ -[bis(2-diphenylphosphinopentyl)phenylphosphine] tris-(1-thio-ß-D-glucopyranosato-S)gold

f. μ -[bis(2-diphenylphosphinohexyl)phenylphosphine] tris-(1-thio-ß-D-glucopyranosato-S)gold

g. μ -[bis(2-diphenylphosphinoethyl)phenyl-

phosphine] tris-(1-thio-α-D-mannopyranosato-S)gold

h. μ-[bis(2-diphenylphosphinoethyl)phenylphosphine] tris-(1-thio-ß-D-galactopyranosato-S)gold

i. μ-[bis(2-diphenylphosphinoethyl)phenylphosphine] tris-(1-thio-D-ribofuranosato-S)gold

EXAMPLE 7

Using the procedure of Example 2, by reacting the appropriate Formula (III) compound wherein m is 0 with the appropriate haloauric acid tetrahydrate, reduced by treatment with thiodiglycol, the following compounds of Formula (III) wherein X is chloro or bromo are prepared; or by reacting the appropriate Formula (III) compound wherein X is chloro with sodium bromide in an appropriate organic solvent such as aqueous ethanol or DMF, the following compounds of Formula (III) wherein X is bromo are prepared; and by reacting the appropriate Formula (III) compound wherein X is chloro or bromo with sodium iodide in an appropriate organic solvent such as acetone, the following compound of Formula (III) wherein X is iodo are prepared:

a. μ-[Tris(2-diphenylphosphinomethyl)phosphino] tetrakis-(chlorogold)

b. μ-[Tris(2-diphenylphosphinopropyl)phosphino] tetrakis-(chlorogold)

c. μ-[Tris(2-diphenylphosphinobutyl)phosphino] tetrakis-(chlorogold)

d. μ-[Tris(2-diphenylphosphinopentyl)phosphino] tetrakis-(chlorogold)

e. μ-[Tris(2-diphenylphosphinohexyl)phosphino] tetrakis-(chlorogold)

f. μ-[Tris(2-diphenylphosphinoethyl)phosphino] tetrakis-(bromogold)

g. μ-[Tris(2-diphenylphosphinoethyl)phosphino] tetrakis-(iodogold)

EXAMPLE 8

Using the procedure of Example 4, by reacting the appropriate thioglucose source with the appropriate Formula (III) compound wherein X is chloro, prepared according to the procedure of Example 2 or 7, the following Formula (III) compounds wherein X is thioglucose are prepared:

a. μ -[Tris(2-diphenylphosphinomethyl)phosphine] tetrakis-(1-thio-ß-D-glucopyranosato-S)gold

b. μ -[Tris(2-diphenylphosphinoethyl)phosphine] tetrakis-(1-thio-ß-D-glucopyranosato-S)gold

c. μ -[Tris(2-diphenylphosphinopropyl)phosphine] tetrakis-(1-thio-ß-D-glucopyranosato-S)gold

d. μ -[Tris(2-diphenylphosphinobutyl)phosphine] tetrakis-(1-thio-ß-D-glucopyranosato-S)gold

e. μ -[Tris(2-diphenylphosphinopentyl)phosphine] tetrakis-(1-thio-ß-D-glucopyranosato-S)gold

f. μ -[Tris(2-diphenylphosphinohexyl)phosphine] tetrakis-(1-thio-ß-D-glucopyranosato-S)gold

g. μ -[Tris(2-diphenylphosphinoethyl)phosphine] tetrakis-(1-thio-α-D-mannopyranosato-S)gold

h. μ -[Tris(2-diphenylphosphinoethyl)phosphine] tetrakis-(1-thio-ß-D-galactopyranosato-S)gold

i. μ -[Tris(2-diphenylphosphinoethyl)phosphine] tetrakis-(1-thio-D-ribofuranosato-S)gold

EXAMPLE 9

Using the procedure of Example 3, by reacting the appropriate Formula (III) compound wherein m is 0 and the appropriate haloauric acid tetrahydrate, reduced by treatment with thiodiglycol, the following compounds of Formula (III) wherein X is chloro or bromo are prepared; or by reacting the appropriate compound of Formula (III) wherein X is chloro with sodium bromide in an appropriate organic solvent, such as aqueous ethanol or DMF, the

following compounds of Formula (III) wherein X is bromo are prepared; and by reacting the appropriate compound of Formula (III) wherein X is chloro or bromo with sodium iodide in an appropriate organic solvent such as acetone, the following compounds of Formula (III) wherein X is iodo are prepared:

a. μ-[1,1,3,5,7,7-hexaphenyl-1,3,5,7-tetraphosphoheptane]-tetrakis(chlorogold)

b. μ-[1,1,5,9,13,13-hexaphenyl-1,5,9,13-tetraphosphotridecane]tetrakis(chlorogold)

c. μ-[1,1,6,11,16,16-hexaphenyl-1,6,11,16-tetraphosphohexadecane]tetrakis(chlorogold)

d. μ-[1,1,7,13,19,19-hexaphenyl-1,7,13,19-tetraphosphononadecane]tetrakis(chlorogold)

e. μ-[1,1,8,15,22,22-hexaphenyl-1,8,15,22-tetraphosphodocosane]tetrakis(chlorogold)

f. μ-[1,1,4,7,10,10-hexaphenyl-1,4,7,10-tetraphosphodecane]tetrakis(bromogold)

g. μ-[1,1,4,7,10,10-hexaphenyl-1,4,7,10-tetraphosphodecane]tetrakis(iodogold)

EXAMPLE 10

Using the procedure of Example 4, by reacting the appropriate thioglucose source with the appropriate Formula (III) compound wherein X is chloro, prepared according to the procedure of Example 3 or 9, the following Formula (III) compounds wherein X is thioglucose are prepared:

a. μ-[1,1,3,5,7,7-hexaphenyl-1,3,5,7-tetraphosphohepatane]-tetrakis(1-thio-ß-D-glucopyranosato-S)gold

b. μ-[1,1,5,9,13,13-hexaphenyl-1,5,9,13-tetraphosphotridecane]tetrakis(1-thio-ß-D-glucopyranosato-S)gold

c. μ-[1,1,6,11,16,16-hexaphenyl-1,6,11,16-tetraphosphohexadecane]tetrakis(1-thio-ß-D-glucopyranosato-

S)gold

d. μ-[1,1,7,13,19,19-hexaphenyl-1,7,13,19-tetraphosphononadecane]tetrakis(1-thio-β-D-glucopyranosato-S)gold

e. μ-[1,1,8,15,22,22-hexaphenyl-1,8,15,22-tetraphosphodocosane]tetrakis(1-thio-β-D-glucopyranosato-S)gold

f. μ-[1,1,4,7,10,10-hexaphenyl-1,4,7,10-tetraphosphodecane]tetrakis(1-thio-β-D-glucopyranosato-S)gold

g. μ-[1,1,4,7,10,10-hexaphenyl-1,4,7,10-tetraphosphodecane]tetrakis(1-thio-D-mannopyranosato-S)gold

h. μ-[1,1,4,7,10,10-hexaphenyl-1,4,7,10-tetraphosphodecane]tetrakis(1-thio-D-ribofuranosato-S)gold

## EXAMPLE 11

## Chloro[1,1,4,7,10,10-hexaphenyl-1,4,7,10-tetraphosphodecane-$P^1$, $P^4$, $P^7$, $P^{10}$] gold(I)

Chloroauric acid tetrahydrate (1.87 g, 4.54 mmol) in water (20 ml) was reduced upon addition of thiodiglycol (3.0 g, 24.6 mmole) in methanol (60 ml)/water (20 ml) at 0°. 1,1,4,7, 10,10-hexaphenyl-1,4,7,10-tetraphosphodecane (TETRAPHOS-I) (0.76 g, 1.13 mmole), obtained from Strem Chemicals, Inc., Newburyport, Massachusetts, in chloroform (60 ml)/methanol (20 ml) was added, and the mixture was stirred for several hours. The resulting precipitate was collected, dissolved in methylene chloride, hexane was added and the solution was cooled. Collection of successive crops gave 1.35 g (84%) of μ-[1,1,4, 7,10,10-hexaphenyl-1,4,7,10-tetraphosphodecane]tetrakis [chloro-gold(I)], which had a melting point (m.p.) of 184-186°.

To a suspension of μ-[1,1,4,7,10,10-hexaphenyl-1,4,7, 10-tetraphosphodecane]tetrakis[chlorogold(I)] (0.5 g, 0.3 mmole), prepared as described above, in chloroform

(25 ml) was added TETRAPHOS-I (.063 g, 0.94 mmole) in chloroform (25 ml). After stirring the mixture for 18 hours at ambient temperature, the solvent was evaporated and the residue dissolved in methanol, filtered, ethyl ether was added, and the mixture was cooled. The resulting precipitate was collected and dried to give 0.37 g (33%) of the named product as a hydrate, m.p. 241-242°.

EXAMPLE 12

Using the procedure of Example 11 to react the appropriate compound of Formula (III) wherein $R^1$ is phenyl, m is 0 and n is 1 with the appropriate compound of Formula (III) wherein $R^1$ is phenyl, n is 1 and m is 1 prepared according to the procedure of Example 1 by using the appropriate bromo- or chloroauric acid hydrate, the following compounds of Formula (I) wherein X is chloro or bromo are prepared; or by reacting the appropriate compound of Formula (I), wherein X is chloro, with sodium bromide in an appropriate organic solvent, such as aqueous ethanol or DMF, the following compounds of Formula (I) wherein X is bromo are prepared; and by reacting the appropriate compound of Formula (I), wherein X is chloro or bromo, with sodium iodide in an appropriate organic solvent, such as acetone, the following compounds of Formula (I) wherein X is iodo are prepared:

a. Chloro[1,1,3,5,7,7-hexaphenyl-1,3,5,7-tetraphosphoheptane-$P^1$, $P^3$, $P^5$, $P^7$]gold(I)

b. Chloro[1,1,5,9,13,13-hexaphenyl-1,5,9,13-tetraphosphotridecane-$P^1$, $P^5$, $P^9$, $P^{13}$]gold(I)

c. Chloro[1,1,6,11,16,16-hexaphenyl-1,6,11,16-tetraphosphohexadecane-$P^1$, $P^5$, $P^9$, $P^{16}$]gold(I)

d. Chloro[1,1,7,13,19,19-hexaphenyl-1,7,13,19-tetraphosphononadecane-$P^1$, $P^7$, $P^{13}$, $P^{19}$]gold(I)

e. Chloro[1,1,8,15,22,22-hexaphenyl-1,8,15,22-tetraphosphodocosane-$P^1$, $P^8$, $P^{15}$, $P^{22}$]gold(I)

f. Bromo[1,1,4,7,10,10-hexaphenyl-1,4,7,10-tetraphosphodecane-$P^1$, $P^4$, $P^7$, $P^{10}$]gold(I)

g. Iodo[1,1,4,7,10,10-hexaphenyl-1,4,7,10-tetraphosphodecane-$P^1$, $P^4$, $P^7$, $P^{10}$]gold(I)

EXAMPLE 13

As a specific embodiment of a composition of this invention, an active ingredient, such as one part of the compound of Example 3 or Example 11, is dissolved in 5 parts of dimethylacetamide and 5 parts of polyethoxylated castor oil and then normal saline solution qs, and is administered parenterally in one dose of 5 $mg/m^2$ to a host animal afflicted with tumor cells sensitive to that compound.

Claims for the Contracting States: BE, CH, DE, FR, GB, IT, LI, LU, NL and SE.

1. A compound of formula (I) :

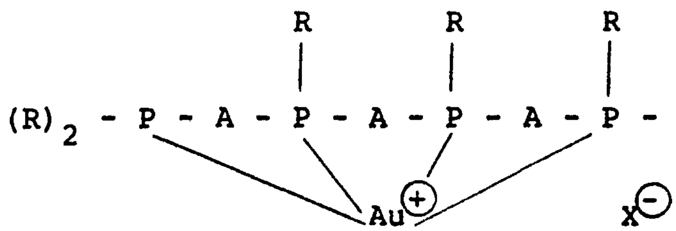

(I)

wherein:

R is phenyl;

$R^1$ is phenyl or $-AP(AuX)(R)_2$;

A is the same and is a straight or branched alkanediyl; chain of from one to six carbon atoms;

n is 0 or 1; and

X is the same and is halo.

2. A compound of formula (II) :

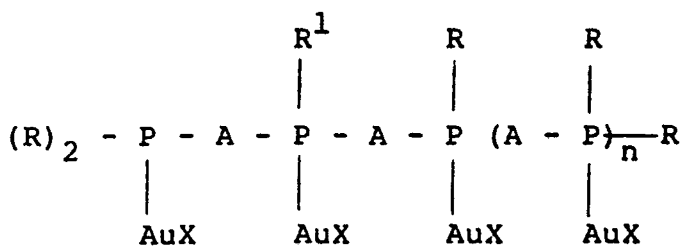

(II)

wherein:

R is phenyl;

$R^1$ is phenyl or $-AP(AuX)(R)_2$;

A is the same and is a straight or branched alkanediyl chain of from one to six carbon atoms;

n is 0 or 1; and

X is the same and is halo or thiosugar;

provided that when n is 1, $R^1$ is phenyl.

3. A compound according to Claim 2 which is μ-[bis(2-diphenylphosphinoethyl)phenylphosphine]tris-(chlorogold), μ-[tris-(2-diphenylphosphinoethyl)phosphine]tetrakis-(chlorogold), μ-[1,1,4,7,10,10-hexaphenyl-1,4,7,10-tetraphosphodecane]-tetrakis(chlorogold), or μ-[1,1,4,7,10,10-hexaphenyl-1,4,7,10-tetraphosphodecane]-tetrakis-(1-thio-ß-D-glucopyranosato-S)gold.

4. A compound according to Claim 1 which is chloro [1,1,4,7,10,10-hexaphenyl-1,4,7,10-tetraphosphodecane-$P^1$, $P^4$, $P^7$, $P^{10}$]gold(I).

5. A pharmaceutical composition which comprises a pharmaceutically acceptable carrier or diluent, and a compound according to Claim 1.

6. A pharmaceutical composition which comprises a pharmaceutically acceptable carrier and a compound of formula (III) :

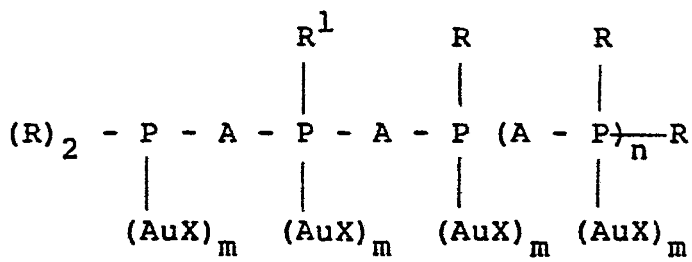

(III)

wherein:

R is phenyl;

$R^1$ is phenyl or $-AP(AuX)(R)_2$;

A is the same and is a straight or branched alkanediyl chain of from one to six carbon atoms;

n is 0 or 1;

m is the same and is 0 or 1; and

X is the same and is halo or thiosugar;

provided that when n is 1, $R^1$ is phenyl.

7. The composition of Claim 6 wherein the compound is
μ-[bis(2-diphenylphosphinoethyl)phenylphosphine]tris-(chlorogold),
μ-[tris(2-diphenylphosphinoethyl)tetrakis(chlorogold),
μ-[1,1,4,7,10,10-hexaphenyl-1,4,7,10-tetraphosphodecane] tetrakis(chlorogold),
μ-[1,1,4,7,10,10-hexaphenyl-1,4,7,10-tetraphosphodecane] tetrakis(1-thio-ß-D-glucopyranosato-S)gold,
bis(2-diphenylphosphinoethyl)phenylphosphine,
tris(diphenylphosphinoethyl)phosphine, or
1,1,4,7,10,10-hexaphenyl-1,4,7,10-tetraphosphodecane.

8. The composition of Claim 5 wherein the compound is chloro[1,1,4,7,10,10-hexaphenyl-1,4,7,10-tetraphospho-decane- $P^1$, $P^4$, $P^7$, $P^{10}$]gold(I).

9. The composition of Claim 5 wherein the composition is in a dosage unit form adapted for parenteral administration.

10. A compound of formula (I), (II) or (III) for use as a therapeutic agent.

11. A compound of formula (I), (II) or (III) for use in inhibiting the growth of animal tumor cells.

12. A process for preparing a compound of the formula:

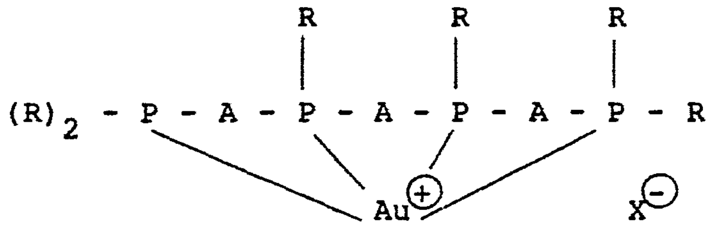

or

Formula (I)

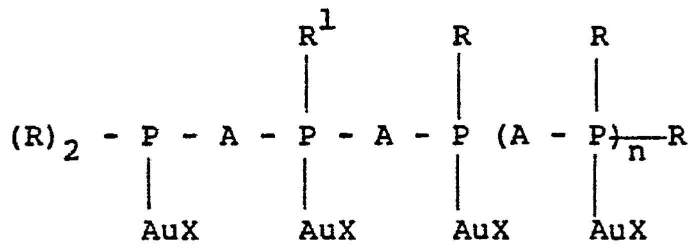

Formula (II)

wherein:

R is phenyl;

$R^1$ is phenyl or $-AP(AuX)(R)_2$;

A is the same as is a straight or branched alkanediyl chain of from one to six carbon atoms;

n is 0 or 1; and

X is the same and is (i) halo for compounds of formula (I) and (ii) halo or thiosugar for compounds of formula (II);

provided that when n is 1, $R^1$ is phenyl; wherein such process comprises:

(a) reacting the appropriate compound of the formula:

$$(R)_2 - P - A - P - A - P\ (A-P)_n - R$$

wherein R, $R^1$, n and A are as described above, with chloroauric acid tetrahydrate or bromoauric acid hydrate reduced by treatment with thiodiglycol to prepare the

compounds of formula (II) wherein X is chloro or bromo, respectively;

(b) reacting the product of part (a), above, with sodium iodide in an appropriate non-reactive organic solvent, such as acetone, to prepare the compounds of formula (II) wherein X is iodo;

(c) reacting the product of part (a), above, wherein X is chloro, with sodium bromide in an appropriate non-reactive organic solvent, such as aqueous ethanol or DMF, to prepare the compounds of formula (II) wherein X is bromo;

(d) reacting the product of part (a), above, wherein X is chloro, with a thiosugar source such as sodium thioglucose, to prepare the compounds of formula (II) wherein X is thioglucose;

(e) reacting the appropriate compound of the formula:

$$(R)_2 - P - A - P - A - P\ (A\text{-}P)_n R$$

wherein R, and A are as described above, $R^1$ is phenyl and n is 1, with the appropriate compound of part (a), above, wherein $R^1$ is phenyl and n is 1, in a non-reactive organic solvent to prepare the compounds of formula (I) wherein X is chloro or bromo;

(f) reacting the product of part (e), above, wherein X is chloro, with sodium bromide in an appropriate organic solvent, such as aqueous ethanol or DMF, to prepare the compounds of formula (I) wherein X is bromo; and

(g) reacting the product of part (e), above, with sodium iodide in an appropriate organic solvent, such as acetone, to prepare the compounds of formula (I) wherein X is iodo.

Claims for the Contracting State AT.

1. A process for preparing a compound of the formula:

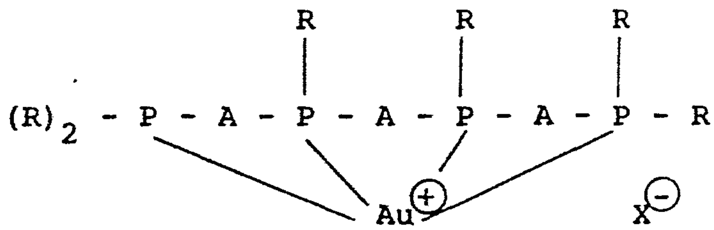

or

Formula (I)

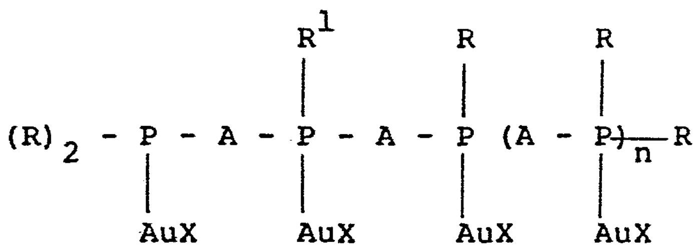

Formula (II)

wherein:

R is phenyl;

$R^1$ is phenyl or $-AP(AuX)(R)_2$;

A is the same and is a straight or branched alkanediyl chain of from one to six carbon atoms;

n is 0 or 1; and

X is the same and is (i) halo for compounds of formula (I) and (ii) halo or thiosugar for compounds of formula (II);

provided that when n is 1, $R^1$ is phenyl;

wherein such process comprises:

(a) reacting the appropriate compound of the formula:

$$(R)_2 - P - A - P - A - P \left(A-P\right)_n R$$

wherein R, $R^1$, n and A are as described above, with chloroauric acid tetrahydrate or bromoauric acid hydrate reduced by treatment with thiodiglycol to prepare the compounds of formula (II) wherein X is chloro or bromo, respectively;

(b) reacting the product of part (a), above, with sodium iodide in an appropriate non-reactive organic solvent, such as acetone, to prepare the compounds of formula (II) wherein X is iodo;

(c) reacting the product of part (a), above, wherein X is chloro, with sodium bromide in an appropriate non-reactive organic solvent, such as aqueous ethanol or DMF, to prepare the compounds of formula (II) wherein X is bromo;

(d) reacting the product of part (a), above, wherein X is chloro, with a thiosugar source such as sodium thioglucose, to prepare the compounds of formula (II) wherein X is thioglucose;

(e) reacting the appropriate compound of the formula:

$$(R)_2 - P - A - P - A - P \, (A-P \overline{)_n} R$$

wherein R, and A are as described above, $R^1$ is phenyl and n is 1, with the appropriate compound of part a, above, wherein $R^1$ is phenyl and n is 1, in a non-reactive organic solvent to prepare the compounds of formula (I) wherein X is chloro or bromo;

(f) reacting the product of part (e), above, wherein X is chloro, with sodium bromide in an approriate organic solvent, such as aqueous ethanol or DMF, to prepare the compounds of formula (I) wherein X is bromo; and

(g) reacting the product of part (e), above, with sodium iodide in an appropriate organic solvent, such as acetone, to prepare the compounds of formula (I) wherein X is iodo.

2. The process of Claim 1 wherein the compound is a compound of formula (II).

3. The process of Claim 2 wherein the compound is μ-[bis(2-diphenylphosphinoethyl)phenylphosphine]tris-(chlorogold).

4. The process of Claim 2 wherein the compound is μ-[tris-(2-diphenylphosphinoethyl)phosphine]tetrakis-(chlorogold).

5. The process of Claim 2 wherein the compound is μ-[1,1,4, 7,10, 10-hexaphenyl-1,4,7,10-tetraphosphodecane]-tetrakis(chlorogold).

6. The process of Claim 2 wherein the compound is μ-[1,1,4,7, 10, 10-hexaphenyl-1,4,7,10-tetraphosphodecane]-tetrakis-(1-thio-ß-D- glucopyranosato-S)gold.

7. The process of Claim 1 wherein the compound is a compound of formula (I).

8. The process of Claim 7 wherein the compound is chloro [1,1,4,7,10, 10-hexaphenyl-1,4,7,10-tetraphospho-decane-$P^1$, $P^4$, $P^7$, $P^{10}$]gold(I).

9. A process for preparing a pharmaceutical composition which comprises admixing a pharmaceutically acceptable carrier or diluent, and a compound of the formula:

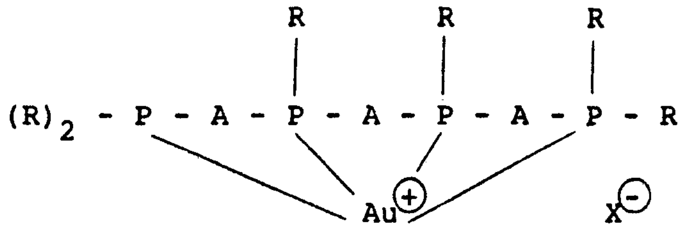

or

Formula (I)

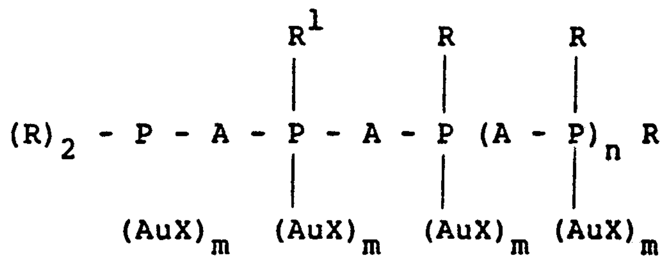

Formula III

wherein:

R is phenyl;

$R^1$ is phenyl or $-AP(AuX)(R)_2$;

A is the same and is a straight or branched alkanediyl chain of from one to six carbon atoms;

n is 0 or 1;

m is the same and is 0 or 1; and

X is the same and is (i) halo for compounds of formula (I) and (ii) halo or thiosugar for compounds of formula (II);

provided that when n is 1, $R^1$ is phenyl.

10. The process of Claim 9 wherein the composition is in a dosage unit form adapted for parenteral administration.